(19) Europäisches Patentamt
European Patent Office
Office européen des brevets

(11) Veröffentlichungsnummer: **0 266 510**
**A2**

# (12) EUROPÄISCHE PATENTANMELDUNG

(21) Anmeldenummer: 87112972.2

(22) Anmeldetag: 04.09.87

(51) Int. Cl.4: C07C 45/72 , C07C 49/293 , B01J 23/10 , B01J 23/06

(30) Priorität: 06.11.86 DE 3637788

(43) Veröffentlichungstag der Anmeldung: 11.05.88 Patentblatt 88/19

(84) Benannte Vertragsstaaten: BE CH DE FR GB IT LI NL

(71) Anmelder: HÜLS AKTIENGESELLSCHAFT
- RSP Patente / PB 15 - Postfach 13 20
D-4370 Marl 1(DE)

(72) Erfinder: Kaufhold, Manfred, Dr.
Jasminweg 20
D-4370 Marl(DE)
Erfinder: Kleine-Homann, Walter, Dr.
Buchenallee 14
D-4408 Dülmen(DE)

## (54) Verfahren zur Herstellung von Methylcyclopropylketon aus Cyclopropancarbonsäure oder ihren Derivaten.

(57) Zur Herstellung von Methylcyclopropylketon leitet man gasförmige Cyclopropancarbonsäure bzw. deren Derivate zusammen mit Essigsäuredämpfen bei Temperaturen von 350 bis 500 °C über einen Ceroxid und/oder Zinkoxid-Festbett-Kontakt. Die Essigsäure und die Cyclopropancarbonsäure bzw. deren Derivate setzt man bevorzugt in Molverhältnissen von 1 bis 20 : 1 ein.

EP 0 266 510 A2

## Verfahren zur Herstellung von Methylcyclopropylketon aus Cyclopropancarbonsäure oder ihren Derivaten

Die Erfindung betrifft ein Verfahren zur Herstellung von Methylcyclopropylketon (1) durch Überleiten von gasförmiger Cyclopropylcarbonsäure (2) oder ihren Derivaten zusammen mit Essigsäuredämpfen über einen Cer-und/oder Zink-Festbett-Kontakt bei erhöhter Temperatur. Als Nebenprodukt erhält man Dicyclopropylketon (3) in geringen Mengen.

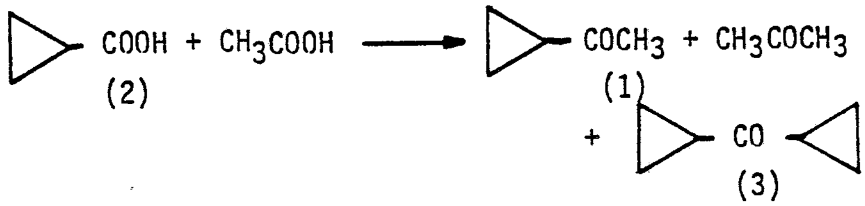

(2) (1) (3)

Es sind zahlreiche Synthesen von (1) in der Literatur beschrieben wie beispielsweise in dem Übersichtsartikel von J. M. Conia, Angew. Chemie 80 (1968), Nr. 15, Seiten 578 bis 585. Die meisten Synthesen benötigen kostspielige Ausgangsstoffe und sind nur für Laborarbeiten geeignet wie z. B. die Simmon-Smith-Reaktion mit ungesättigten Ketonen, bei der das sogenannte Zink-Kupfer-Paar zusammen mit dem teuren Methylenjodid eingesetzt wird.

Eine technisch gebräuchliche Synthese ist in Org. Syntheses Coll., Vol. IV (1963), Seite 597 beschrieben:
Sie geht vom alpha-Acetyl-gamma-butyrolaceton (4) aus, das mit Salzsäure zum 5-Chlor-2-pentanon (5) unter Kohlendioxid-Abgabe umgesetzt wird, das anschließend mit Natronlauge eine Cyclisierungsreaktion zum Methylcyclopropylketon eingeht:

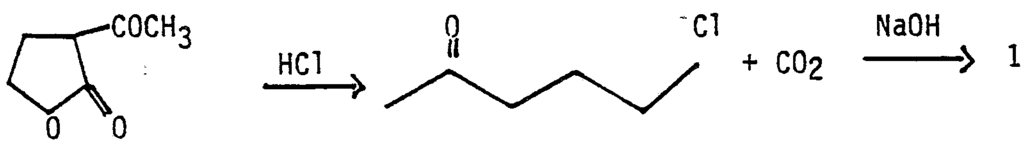


4 5

Das Problem bei dieser zweistufigen Synthese ist, daß man von einer teuren Chemikalie ausgehen muß, da (4) nur in schlechten Ausbeuten mit hohem technischen Aufwand herstellbar ist (Org. Synth. Coll. Vol. IV (1963), S. 597).

Alle bekannten Verfahren benötigen somit teure Chemikalien und sind zudem zum Teil technisch nicht realisierbar. Wünschenswert wäre ein Verfahren, bei dem man von einem leicht zugänglichen und darum preisgünstigen Stoff ausgehen und diesen in einem katalytischen Verfahren ohne hohen Chemikalienverbrauch zum Methylcyclopropylketon umsetzen könnte.

An einem derartigen Verfahren besteht ein großes Interesse, weil dieses Keton Rohstoff für Pflanzenschutz-und Pharma-Produkte ist.

Die sich hieraus ergebende Aufgabe wird erfindungsgemäß entsprechend den Angaben der Patentansprüche gelöst.

Überraschenderweise erhält man beim Überleiten von gasförmiger Cyclopropancarbonsäure oder ihren Derivaten mit Essigsäuredämpfen über einen Festbettkatalysator, der die Oxide von Cer und/oder Zink enthält, bei erhöhter Temperatur Methylcyclopropylketon in guten Ausbeuten.

Als Derivate der Cyclopropancarbonsäure sind Ester der niederen Alkohole wie Methanol, Ethanol, Propanole und Butanole geeignet. Bevorzugt setzt man die Cyclopropancarbonsäure ein. Essigsäure und die Cyclopropancarbonsäure bzw. ihre Derivate setzt man im allgemeinen in Molverhältnissen von 1 : 1 bis 20 : 1 ein, vorzugsweise von 3 : 1 bis 15 : 1 ein, insbesondere von 5 : 1 bis 10 : 1 ein.

Geeignete Festbettkatalysatoren sind ZnO und/oder CeO-dotierte Kontakte, bevorzugt die CeO-dotierten auf $Al_2O_3$. Die Festbettkatalysatoren enthalten im allgemeinen 5 bis 25 % ZnO und/oder CeO, bevorzugt 10 bis 20 %, insbesondere 12 bis 18 %. Der eingesetzte $Al_2O_3$-Träger hat im allgemeinen einen $Al_2O_3$-Gehalt von 90 bis 99 %. Er wird bevorzugt in Form von zerkleinerten Strängen und einem Durchmesser von 1 bis 2 mm eingesetzt. Die zerkleinerten Stränge haben vorzugsweise ein spezifisches Porenvolumen von 0,3 bis 1,0 $cm^3/g$.

Die Umsetzung erfolgt bei Temperaturen von 350 bis 500 °C, bevorzugt von 400 bis 460 °C.

Man erhält das Methylcyclopropylketon bei guten Umsätzen von über 99 % in guter Selektivität von mehr als 70 %.

Dieser Befund ist deswegen so überraschend, weil bekanntlich Cyclopropylverbindungen thermisch relativ unbeständig sind und sich vor allem in Gegenwart von Katalysatoren leicht zu den entsprechenden geradkettigen Verbindungen umlagern. Eine weitere bekannte thermische Umlagerung speziell des Methylcyclopropylketons führt nach Arbeiten von A. T. Cocks und K. W. Egger (s. Journ. Soc., Perkin Trans., II, 1973, 2, 197-99, 199-203) zum 2.3-Dihydro-5-methyl-furan (6):

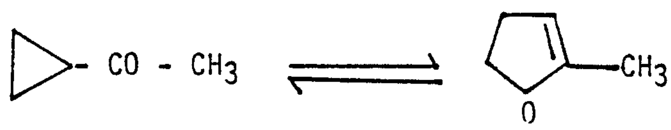

(1) (6)

Ferner war anzunehmen, daß die Essigsäure hauptsächlich Aceton und die Cyclopropancarbonsäure hauptsächlich Dicyclopropylketon liefern würde. Es zeigte sich aber, daß man die Reaktion durch geeignete Überschüsse an Essigsäure in die gewünschte Richtung lenken kann.

Ein Vorteil der Erfindung ist der geringe Verbrauch an Chemikalien, die außerdem billig sind, und daß der Reaktoraustrag ohne weitere Arbeitsvorgänge direkt durch Destillation aufgearbeitet werden kann.

## Beispiele

0,2 l des entsprechenden Kontaktes (s. Tabelle) werden in einem beheizbaren Quarzrohr von 0,5 m Länge und 25 mm Durchmesser vorgelegt. Unter Zugabe von Stickstoff wird der jeweilige Kontakt auf die gewünschte Temperatur erwärmt (s. Tabelle). Die Temperaturerfassung erfolgt mit Hilfe eines Widerstandsthermometers, das sich in einem zentrierten Quarzrohr von 6 mm Durchmesser befindet und sich dort je nach Anforderung axial in die gewünschte Position verschieben läßt.

Das Gemisch bestehend aus Essig-und Cyclopropancarbonsäure wird in dem mit VA-Ringen gefüllten oberen Teil des Reaktors verdampft und danach über den Katalysator geleitet. Der kondensierte Reaktoraustrag wird anhand von GC-Anlaysen ausgewertet.

Bei der destillativen Aufarbeitung wird bei Normaldruck an einer 0,5 m langen mit Mulbifil-Füllkörpern gefüllten Glaskolonne zunächst Aceton in 99,9 %iger Reinheit abdestilliert. Dann gibt man Hexan zu und kreist damit Wasser aus. Nach dem Auskreisen des Wassers destilliert man das zugesetzte Hexan in einem Siedebereich von 61 bis 70 % ab.

GC-Anlayse der Hexanfraktion:
Hexan 98 %
Methylcyclopropylketon 0,5 %
Essigsäure 0,5 %

In einem Siedebereich von 70 bis 110 °C fällt ein Zwischenlauf an, der nach gaschromatographischer Analyse zu ca. 15,5 % aus Methylcyclopropylketon und zu ca. 84 % aus Wasser besteht.

In einem Siedebereich von 110 bis 112 °C erhält man das Methylcyclopropylketon in 98,5 %iger Reinheit.

| Beispiel | Mol-Verhältnis Essig-/Cyclopropan Carbonsäure | LHSV (ml Säuren/ ml Kontakt x h) | Kontakt | Reaktions- temperatur ( C) | Umsatz (%) | Selektivität (%) |
|---|---|---|---|---|---|---|
| 1 | 4 | 0,9 | I | 465 | 99 | 75 |
| 2 | 10 | 0,5 | I | 400 | 70 | 70 |
| 3 | 13 | 0,6 | I | 440 | 80 | 70 |
| 4 | 13 | 0,5 | II | 425 | 88 | 42 |
| 5 | 3 | 0,25 | II | 390 | 58 | 67 |

Katalysator I : 12 % CeO auf $AL_2O_3$

Katalysator II: 18 % ZnO auf $Al_2O_3$

Das in den Katalysatoren eingesetzte $Al_2O_3$ hat einen $Al_2O_3$-Gehalt von 91 %. Der $Al_2O_3$-Träger wird in Form von zerkleinerten Strängen mit einem Durchmesser von 1,6 mm eingesetzt. Die zerkleinerten Stränge haben eine Schüttdichte von 0,84 g/cm³ und ein spezifisches Porenvolumen von 0,41 cm³/g.

**Ansprüche**

1. Verfahren zur Herstellung von Methylcyclopropylketon,
dadurch gekennzeichnet,
daß man gasförmige Cyclopropancarbonsäure bzw. deren Derivate zusammen mit Essigsäuredämpfen bei Temperaturen von 350 bis 500 °C über einen Ceroxid und/oder Zinkoxid-Festbett-Kontakt leitet.

2. Verfahren nach Anspruch 1,
dadurch gekennzeichnet,
daß man die Umsetzung bei Temperaturen von 400 bis 460 °C, durchführt.

3. Verfahren nach Anspruch 1 und 2,
dadurch gekennzeichnet,
daß man die Essigsäure und die Cyclopropancarbonsäure bzw. deren Derivate in Molverhältnissen von 1 bis 20 : 1 einsetzt.

4. Verfahren nach einem der Ansprüche 1 bis 3,
dadurch gekennzeichnet,
daß man die Essigsäure und die Cyclopropancarbonsäure bzw. deren Derivate in Molverhältnissen von 3 bis 15 : 1 einsetzt, bevorzugt von 5 bis 10 : 1

5. Verfahren nach einem der Ansprüche 1 bis 4,
dadurch gekennzeichnet,
daß man als Festbettkatalysatoren mit ZnO und/oder CeO dotiertes $Al_2O_3$ einsetzt.